Europäisches Patentamt

European Patent Office

Office européen des brevets

(11) Publication number: 0 499 765 A1

# EUROPEAN PATENT APPLICATION

(21) Application number: **91610009.2**

(22) Date of filing: **19.02.91**

(51) Int. Cl.⁵: **G01N 33/12**, A22B 5/00, G01N 29/18

(43) Date of publication of application:
**26.08.92 Bulletin 92/35**

(84) Designated Contracting States:
**AT BE CH DE DK ES FR GB GR IT LI LU NL SE**

(71) Applicant: **SLAGTERIERNES FORSKNINGSINSTITUT Maglegaardsvej 2, P.O. Box 57 DK-4000 Roskilde(DK)**

(72) Inventor: **Hjelmroth, Hans-Erik Kjaersvej 123 DK-4220 Korsoer(DK)**

(74) Representative: **Vingtoft, Knud Erik et al Plougmann & Vingtoft Sankt Annae Plads 11 P.O. Box 3007 DK-1021 Copenhagen K(DK)**

(54) **A method and apparatus for determining and/or evaluating quality characteristics of a body of meat material.**

(57) In order to determine and/or evaluate quality characteristics of a body of meat material (32) at least one probe (10) containing means (24) for emitting mechanical vibrations is plunged into the meat body so as to position the vibration emitting means (24) therein. The mechanical vibrations emitted by the emitting means, which may, for example, be a mechanical transducer (24), are detected by detecting means, which may also be the transducer (24). Quality characteristics of the meat body may then be determined and/or evaluated on the basis of signals generated in response to mechanical vibrations detected by the detecting means (24). As the mechanical vibrations may be emitted from a desired location inside the meat body (32) within the tissue structure to be examined, and as the window of the emitting and detecting means or transducer (24) may be arranged in good frictional or abutting contact with the meat substance of the body, the results obtained may be substantially improved compared with known methods.

Fig. 3

The present invention provides a method of determining and/or evaluating quality characteristics of a body of meat material, such as a carcass or part of a carcass of a pig or an ox, a fish, a slaughtered poultry, a body of processed meat, such as minced meat, a piece of pickled and/or smoked meat, such as a ham, or the like. It is envisaged that the method according to the invention could also be used for examining meat quality characteristics of a living animal.

Methods and apparatuses for determining and evaluating tissue structure in a living human body for diagnostic purposes and for determining and/or evaluating tissue structure or the meat thickness of and/or fat of a living animal body or an animal carcass by means of ultrasonic vibrations are well known in the art. Thus, methods and apparatuses in which ultrasonic transducer means are arranged outside the body to be examined or within a natural inner cavity of the body are disclosed for example in Us patents Nos. 4,928,699, 4,417,583, 4,756,313, 4,359056 and 4,785,817. In these known methods and apparatuses the evaluation and determination of the various types of the tissue layers located inside the outer skin surface of the body are based on i.a. the delay and intensity of ultrasonic vibrations reflected by the various tissue layers. Because of "false echoes", for example from the skin surface and tissue structures immediately below the outer skin surface, the reflected "ultrasonic picture" received by the transducer means may be blurred. This is especially true when such known method is used for evaluating and classifying pig carcasses with outer skin surfaces which have been exposed to burners or to a similar heat treatment, for example in a singeing furnace.

Furthermore, Danish patent No. 120,003 discloses a method of determining the thickness of fat and meat layers in pig carcasses. In this known method a probe containing means for emitting light and means for determining reflected light is plunged into the carcass. The depth of penetration of the probe is measured by mechanical means, and the reflected light measured indicates whether the tissue immediately around the probe is fat or meat.

The present invention provides a method enabling an improved and more accurate evaluation and/or determination of various quality characteristics of the meat material. Thus, the present invention provides a method of determining and/or evaluating quality characteristics of a body of meat material, said method comprising plunging at least one probe containing means for emitting mechanical vibrations into said body so as to position the vibration emitting means within said body, emitting mechanical vibrations within said body by the vibration emitting means, detecting mechanical vibrations emitted by the emitting means by the detecting means, generating detection signals in response to mechanical vibrations detected by the detecting means, and determining and/or evaluating quality characteristics of said body on the basis of signals detected.

Because the mechanical vibrations may be emitted from a desired location inside the body within the tissue structure to be examined, and because the window of the transducer is in good frictional or abutting contact with the meat substance, the results obtained may be substantially improved compared with known methods.

The good contact is obtained because the contact surfaces are fresh and due to the presence of meat juice. If the probe was inserted in a natural cavity the ultrasonic vibrations would be damped by the stiff surfaces of the cavity and a similar good contact could be obtained only by introducing a fluid substance into the cavity. However, this would be undesirable for hygienic reasons.

The mechanical vibrations emitted by the emitting means inside the meat body may be detected by suitable detecting means arranged at one or more spaced locations outside the meat body, preferably in close contact with the outer surface thereof. In the preferred embodiment, however, at least one detecting means is also positioned inside the body. These detecting means may then be arranged within one or more transversely spaced probes. Vibration emitting means and the vibration detecting means may be arranged within each probe. In the preferred embodiment, however, only one or a few probes have vibration emitting means.

The mechanical vibrations may be emitted with a frequency within the ultrasonic range. Frequencies within the range of 40 kHz to 20 MHz are preferred. Alternatively, the frequency of the vibrations may be even lower so that the vibrations are audible. The vibrations may be generated by means of any kind of vibration generating means which may be arranged within the probe or probes plunged into the body of meat material. Alternatively, the vibration generating means may be positioned outside the body of meat material and the probe may contain means for transmitting the vibrations axially through the probe to a position within the body of meat material from which position the vibrations are emitted.

When the mechanical vibrations emitted by the vibration emitting means are ultrasonic vibrations, the means for generating the ultrasonic vibrations may be an ultrasonic transducer of any suitable type, and the means for generating and the means for detecting the ultrasonic vibrations may be the same transducer or different transducers. By way of example, the ultrasonic transducer used for generating and/or detecting the ultrasonic vibrations may comprise any of the following types of transducers: Piezoelectric transducers, magnetostrictive transducers, electromagnetic vibrators, Hartmann whistles, modified Hartmann whistles and counterbalanced mechanical vibration generators.

The mechanical vibrations may be emitted substantially in the longitudinal direction of the probe. In the preferred embodiment, however, the vibrations emitted are directed transversely to the longitudinal direction of the probe containing the emitting means. The probe may have any suitable cross-sectional shape, for example circular or polygonal, such as triangular, square or hexagonal. The probe may then define transversely directed windows through which the mechanical vibrations are emitted. When the probe has a polygonal cross-sectional shape, such as the shape of a regular polygon, a window may be defined in each side surface of the probe. The mechanical vibrations may be emitted in any suitable transverse direction defining an acute angle with the longitudinal axis of the probe. However, the mechanical vibrations are preferably emitted substantially at right angles to the longitudinal direction of the probe. The mechanical vibrations may be emitted from the emitting means through an angle, the vertex of which is positioned at the emitting means, and this angle may, for example, be defined in a plane extending through the longitudinal axis of the probe, which contains the emitting means. Alternatively, the mechanical vibrations may be emitted by the emitting means as a band-like beam along a plane extending through the longitudinal axis of the probe, which contains the emitting means.

When it is desired to scan a volume of the body of meat material surrounding the probe the emitting means may be rotated about the longitudinal axis of the probe, which contains the emitting means, while mechanical vibrations are emitted and detected continuously or intermittently during such rotation. It should be understood that the emitting means emitting the mechanical vibrations transversely to the longitudinal axis of the probe could be rotated within the probe and in relation thereto, or the emitting means could be fixedly mounted within the probe and the probe could be rotated together with the transmitting means. Preferably, in the latter case the probe would also contain detecting means, which are also rotated together with the probe. Alternatively or additionally, the vibration emitting means may be moved in the longitudinal direction of the probe while vibrations are emitted and detected continuously or intermittently. In principle, the vibration emitting means could be moved longitudinally in relation to the probe and possibly rotated at the same time so as to define a helical path. In the preferred embodiment, however, vibrations are emitted and detected while the probe is plunged into the meat body and possibly rotated at the same time.

Two or more probes arranged in spaced relationship may be plunged into the meat body. Vibration emitting means may then be arranged in both or more of the probes and detecting means may be arranged outside the meat body and/or in one or more of the probes. In the preferred embodiment, however, the emitting means are arranged in only one of said probes, and the remaining probe or probes may then each contain vibration detecting means. In an alternative embodiment a pair of spaced probes each containing detecting as well as emitting means are used. Vibrations may then be emitted from one of the probes and detected by the other. Thereafter, the functions of the probes may be reversed, so that vibrations are emitted from said other probe and detected by said one probe. Furthermore, mechanical vibrations may be emitted into the body by emitting means arranged outside the body, if desired.

The mechanical vibrations may be emitted substantially continuously or as sequences of short pulse trains. The vibrations may be emitted at substantially one predetermined frequency or may be emitted at a range of frequencies, such as a frequency band. In the latter case, the frequency or frequencies of the vibrations may be increased or decreased continuously or stepwise.

Various quality characteristics of the meat body into which the probe is plunged influence the manner in which the mechanical vibrations propagate through the meat body. Thus, the quality characteristics, such as the thickness of meat layers, the thickness of fat layers, fleshiness, "fat marbling", or the meat/fat pattern, the structure of the meat fibres, the structure of connective tissue, the water content, the amount of PSE- and DFD-meat, and the ability of the meat body to bind the meat juice therein may influence fundamental physical quantities i.a. velocity of vibration propagation, vibration damping, change of impedance, duration of echoes, and/or characteristics of such echoes of the mechanical vibrations. These physical quantities are different for meat, fat and water and a determination or an evaluation of one or more of the quality characteristics may be based on the determination of one or more of said physical quantities.

The method according to the invention renders it possible to make simple and reliable measurements rather quickly. Such simple measurements may, for example, include measurement of the velocity of the vibration propagation in the meat body between a pair of spaced probes positioned in the meat body, and/or measurement of the impedance of the tissue by using one or more probes plunged into the tissue.

It is also possible to make a more complex measurement by measuring an array of quantities i.a. the intensities of the detected vibrations at one or more detecting means or transducers as a function of time, the positions (such as the depth of probe penetration) of the generating and/or the detecting means as a function of time, the temperature at the locations of the generating and/or detecting means inside the meat tissue as a function of time, the frequency spectra of the detected vibrations at one or more detections means or transducers, the reference frequency spectrum transmitted in water by the generating means (the transmitter transducer) and detected in water by the detecting means, and/or the thrust used to plunge the probe into the meat tissue.

The said complex measurements may initially be made on a large number of meat pieces for which the quality characteristics are known and the data obtained from these measurements and information about the correlated quality characteristics may be stored and analyzed in a computer establishing correlation between the array of detected data and the known quality characteristics. The correlation can be expressed by using a multivariable algorithm or be established by using neural networks recognizing structures.

After such initial measurements of a large number of meat bodies the basis for the multi-algorithm or the neural networks is established, and measurements of meat pieces or meat bodies with unknown quality characteristics and determination of these characteristics for the individual piece of meat may now be made with a high degree of correlation on the basis of the signals detected.

Methods are known according to which quality characteristics are determined for a large number of meat bodies or meat pieces in such a way that the overall statistic description is rather good, but there may be large fluctuations for the individual meat body or meat piece. Thus in previous methods the correlation coefficient is good for description of the whole group of meat bodies, but may be rather poor for the individual meat body. By making the complex measurement according to the invention, it is possible to determine the quality characteristics for the individual meat body or meat piece and thereby obtain a rather good individual statistic description of for the individual meat body or meat piece. Thus, a good correlation coefficient for the statistic description of the individual meat body or meat piece can be obtained.

As the windows of the transducers are in close frictional contact with the meat substance, some of the detected signals obtained by using the method according to this invention are very sensitive to the characteristics of the tissue structure. When the vibrations are emitted through a window, multireflections are generated at the window. The structure of these multireflections is very sensitive to the substance located outside and being in frictional contact with the window. Surface vibrations are also very sensitive to the substance just outside the window of a transducer. Detailed information can be extracted from the data by analyzing the damping of these multireflections and of these surface vibrations and by performing spectral analyzing.

It is possible to cut a passage or pocket in the meat body by means of a special cutting tool and to insert the probe containing the vibration emitting means in such a pocket. In the preferred embodiment, however, the probe is shaped so as to serve as a cutting tool. Thus, the probe may have a free end which is pointed or defines a cutting edge for cutting a pocket or passage in the meat body when the probe is driven into the meat body under the influence of an axially directed manual or mechanical thrust. Obviously, the thrust necessary to insert the probe in the meat body is dependent on the ultimate strength of the meat tissue to be pierced. This ultimate strength is also an indication of the type of tissue and of the tenderness of the meat. Therefore, the insertion thrust is preferably continuously or intermittently determined when the probe is plunged into the meat body, and the information obtained may be used as a basis for the evaluation of the quality characteristics of the meat body. As the various physical values measured may be dependent on the temperature of the meat body, such temperature is preferably also measured and taken into account.

In order to be able to make an accurate evaluation of the quality of the meat body it is also important to know exactly at which locations the respective measurements are being made. Therefore, the depth of penetration of the emitting means and/or detecting means into the body of meat material is preferably determined, and the vibrations detected may then be interrelated to the determined depth of penetration. When measurements are being made while the probe is moved axially and plunged into the meat, the depth should be measured continuously or intermittently. If, however, mechanical vibrations are being emitted in only one location of the body, only the depth of such location need be measured. Similarly, when the probe and/or the emitting means are being rotated it is important to continuously or intermittently determine the rotational position of the probe or probes and to interrelate such determined position to the vibrations detected.

The present invention also provides an apparatus for determining and/or evaluating quality characteristics of a body of meat material, said apparatus comprising at least one probe to be plunged into said body and including means for emitting mechanical vibrations, means for detecting mechanical vibrations emitted by the emitting means, and means for generating detection signals in response to mechanical vibrations detected by the detecting means.

The apparatus may further comprise means for processing and/or registering said detection signals. Such processing means may comprise electronic processing circuits, such as computers. As an example, the processing means may comprise neural networks which may compare the structure of the signals received with stored information about structures for a large plurality of known tissue structures.

The invention will now be further described with reference to the drawings, wherein

Fig. 1 is a side view of an ultrasonic probe device according to the invention,

Fig. 2a is a side view and partially sectional view of the probe of Fig. 1 shown in an enlarged scale,

Fig. 2b is a side view of the free end portion of the probe shown in Figs. 1 and 2a in an enlarged scale, and

Figs. 3-8 diagrammatically illustrate various embodiments of the apparatus according to the invention during use.

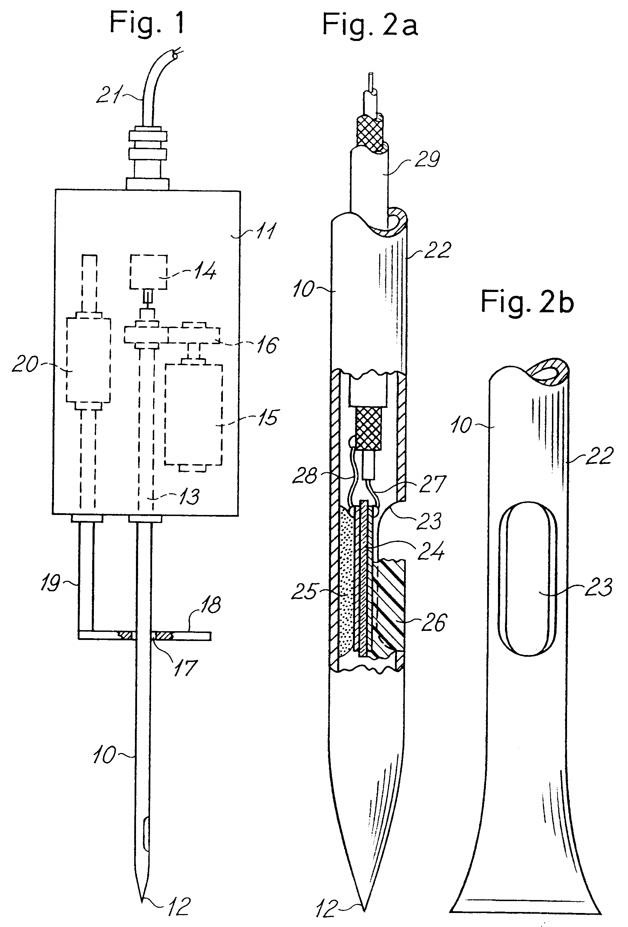

The probe device shown in Fig. 1 comprises a probe 10 extending from a housing 11. The probe 10 has a free end 12, which is pointed or defines a cutting edge. In the embodiment shown the probe 10 has a circular cross-section, which may, however be polygonal, such as triangular or square, if desired. The probe 10 has an inner part 13 extending into and rotatably mounted in the housing 11. The inner end of the inner part 13 of the probe is in force transmitting contact with thrust measuring means, such as a pressure transducer 14. The housing 11 also contains a motor 15, such as an electric step motor, for continuous or stepwise rotation of the probe 10 through driving means 16, such a pair of meshing gears mounted on the driving shaft of the motor and on the inner part 13 of the probe, respectively. The probe 10 extends through an opening 17 defined in a contact plate 18 extending transversely to the probe 10. The contact plate 18 is fixed to a mounting rod 19, which extends into the housing 11 and which is mounted so as to be axially displaceable in relation to the housing between an extended position in which the contact plate 18 is located adjacent to the pointed end 12 of the probe 10, and a retracted position in which the contact plate 18 is located closer to the housing 11. The mounting rod 19, which is substantially parallel to the probe 10, extends through a position detecting means 20 mounted stationarily within the housing 11 for detecting the position of the contact plate 18 in relation to the probe 10, namely the distance between the pointed end 12 of the probe 10 and the adjacent surface of the contact plate 18. The position detecting means may, for example be a digital or analog device, such as a potentiometer, and the mounting rod 19 may be biassed towards its extended position, for example by spring means, not shown. An electric cable 21 may supply electric power to the electric devices contained in the housing 11 and may be used for transferring detecting and measuring signals from detecting and measuring means in the probe 10 and the housing 11.

As shown in Figs. 2a and 2b the probe 10 may comprise an outer substantially cylindrical jacket 22, which is preferably made from metal, and which is pointed at its free end. An elongated opening or window 23 is formed in the jacket 22 adjacent to the pointed free end 12. An ultrasonic transducer 24 is mounted within the jacket 22 opposite to the window 23. The side surface of the transducer 24 remote from the window 23 is supported by a layer 25 of a vibration damping material, and the opposite side surface of the transducer 24 adjacent to the window 23 is in contact with a layer 26 of a vibration transmitting material, such as ceramic, metallic or polymer material, for example ARALDITE. The vibration transmitting material also fills the window 23 so that the outer surface of the material 26 flushes with the outer cylindrical surface of the jacket 22.

The ultrasonic transducer 24 is preferably of the type which may generate as well as detect ultrasonic vibrations, and electric signals may be supplied to and from the transducer 24 through electric conductors 27 and 28 forming part of an electric cable 29.

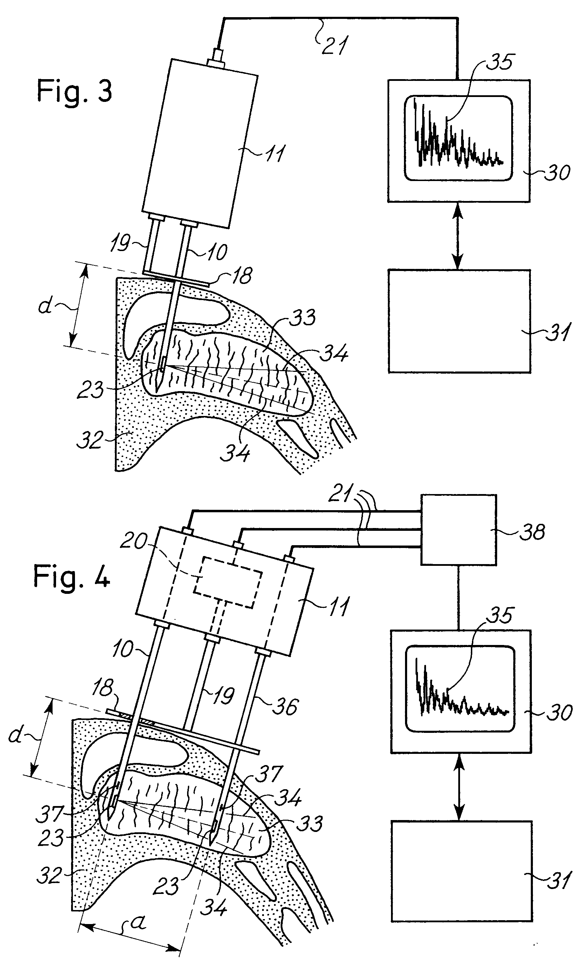

As shown in Fig. 3, the cable 21 may be connected to a registering and display device 30 which may receive signals from and cooperate with a computer device 31. In Fig. 3, the probe 10 has been plunged into a meat body, which may, for example, be a rib of beef including a fillet 33. Alternatively, Fig. 3 may show the back of a pig. When the probe 10 is plunged into the meat body 32 by applying a manual or mechanical pressure to the housing 11, the varying axial thrust applied to the probe 10 may be detected by the pressure transducer 14 and interrelated to the depth of penetration "d" registered by the position detecting means 20. Measuring signals generated by the transducer 14 and by the position detecting means 20 may be transmitted to the computer device 31 for further processing. When the free end portion of the probe 10 with the window 23 has been positioned at a desired location within the fillet 33 the ultrasonic transducer 24 may be caused to generate ultrasonic vibrations which are emitted through the window 23 as indicated by lines 34, Fig. 3. Reflected ultrasonic vibrations may be detected also by the transducer 24, and detection signals received by the computer device 31 from the transducer 24 may be processed, rectified and displayed by the display device 30. The displayed signals 35 shown in Fig. 3 indicate a pronounced "marbled" meat structure, which means that the meat of the fillet contains a plurality of fat inclusions. If desired, vibrations may be emitted and detected while the probe is inserted ind and/or retracted from the meat body.

Fig. 4 illustrates a situation similar to that illustrated in Fig. 3, and similar parts have been designated the same reference numerals. In Fig. 4 a modified embodiment of the apparatus according to the invention is being used for determining the meat quality of the back of a pig carcass or the fillet 33 of rib of beef 32. The probe device shown in Fig. 4 comprises two substantially parallelly extending probes, namely the probe 10 containing ultrasonic vibration emitting means, such as an ultrasonic transducer, and a second probe 36 containing vibration detecting means, which may also be an ultrasonic transducer. At least one of the probes 10 and 36 may contain a temperature sensor 37 for sensing the temperature of the meat body 32. In Fig. 4, the contact plate 18 contains openings for receiving and guiding both of the probes 10 and 36, and as previously described the plate 18 is displaceable in order that its position indicates the distance "d" from the outer surface of the meat body 32 to the window 23 of the probes. A unit 38 may receive signals from the detecting means in the probe 36, the temperature sensor 37, possible thrust measuring transducers 14, and the position detecting means 20 and transmit electric signals to the ultrasonic transducer 24 in the probe 10. The signals received by the unit 38 may be further processed in the computer device 31 and registered and/or displayed by the registering and display device 30.

By using two probes placed in predetermined spaced relationship it is possible to determine i.a. the velocity of vibration propagation through the meat tissue and the damping of the transmitted vibration and thereby, it is possible to determine quality characteristics for the meat body in a simple manner. When vibrations are transmitted into a meat body with "fat marbling", a number of reflections are generated at the interface between meat and fat and thus, the structure of the detected intensity spectrum is an indicator of the amount of fat contained in the meat body and of the structure of the fat inclusions in the meat. The detected spectrum is analyzed in the computer. Multireflections are generated at the transducer window of the probes and the damping of these is very dependent on the kind of material placed in close contact with the window. The computer compares an actual measurement with information based on a great number of previous similar measurements, whereby one or more quality characteristics of the meat body may be determined. When the transducer emits vibrations within a range of frequencies the computer can also make a spectral analysis of the frequency spectrum and thereby deduce specific information about the tissue structure.

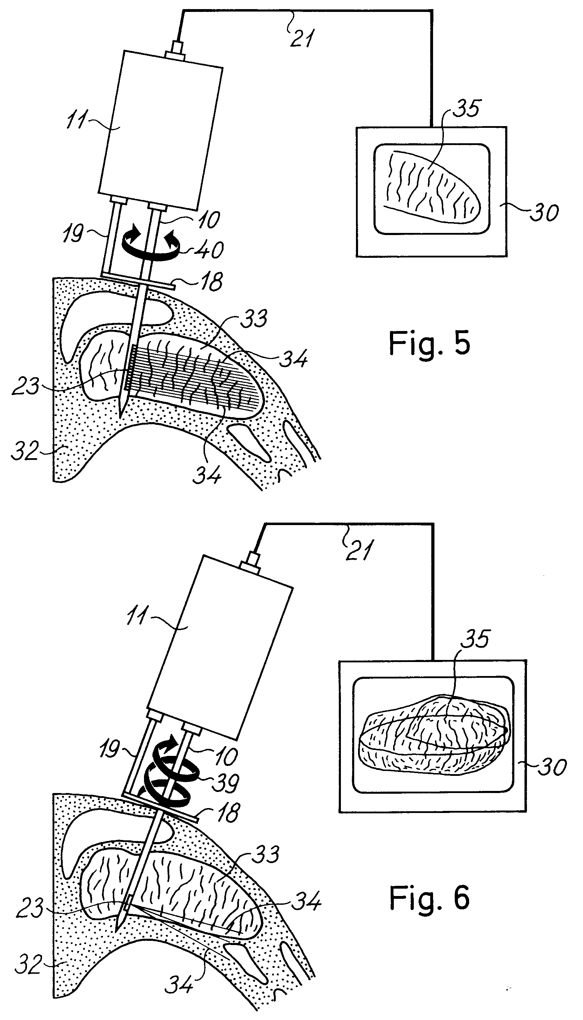

Fig. 5 illustrates a situation similar to that shown in Fig. 1. Only one probe 10 is used and this probe contain an ultrasonic transducer which may be used not only for generating, but also for detecting ultrasonic vibrations. The transducer may be a linear array-scanner forming a band-like beam of ultrasound as indicated by the lines 34. The device 30 may be a detecting, registering and display device for the ultrasonic scanner and may also contain a registering and calculating unit for calculating the signals of fat or tallow.

When the probe 10 has been plunged into the back of a pig carcass or the rib of beef 32 so that the window 23 is positioned at a desired location within the fillet 33, the depth of penetration is detected by the detecting means 20, Fig. 1. Now, the probe 10 may be rotated continuously or stepwise by means of the motor 15, whereby the contour of the fillet 33 as well as the fat distribution or pattern in the fillet may be registered and displayed by the device 30.

In Fig. 6, which is similar to Fig. 5, measurements are being made while the probe 10 is plunged into and moved axially in relation to the meat body 32. At the same time the probe 10 is rotated continuously or stepwise by means of the motor 15, Fig. 1, whereby the window 23 is moved along a helical path as indicated by the helical arrow 39 in Fig. 6, whereby it is possible to obtain signals from which information about i.a. the volume of muscles may be obtained. The probe device may or may not have position detecting means 29 comprising a contact plate 18 and a mounting rod 19. When the apparatus is used as illustrated in Fig. 6 it is possible to obtain "three dimensional information" about the tissue structure around the probe 10, and a projection 35 of a three-dimensional illustration of the fillet 33 may be displayed by the display device 30.

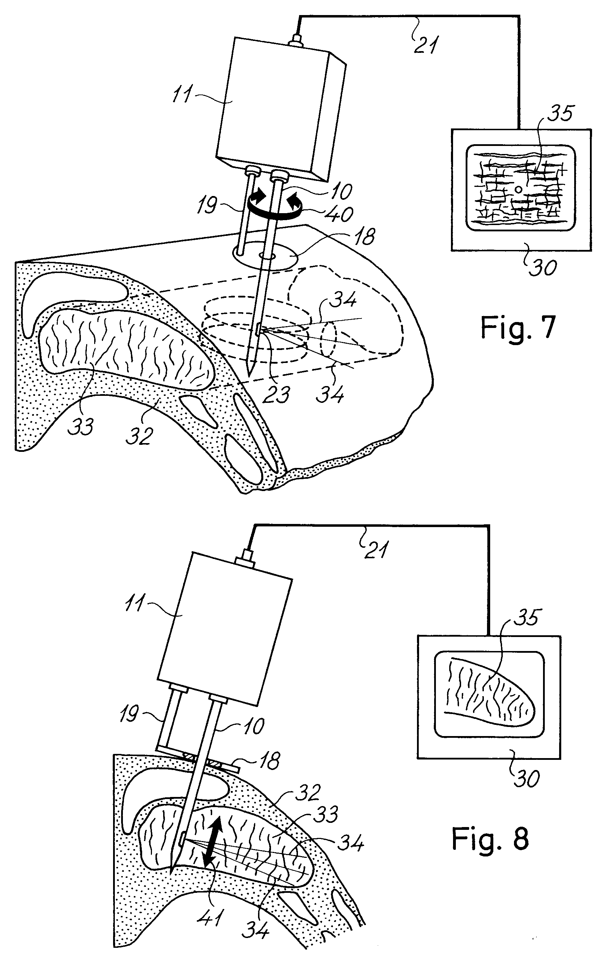

Fig. 7 illustrates a measurement procedure corresponding to that illustrated in fig. 5. However, in fig. 7 the band-like beam of ultrasonic vibrations has been replaced by an angular or conical beam as indicated by the lines 34. As indicated by an arrow 40 the probe 10 may be rotated continuously or intermittently, whereby the tissue structure of the fillet portions around the probe 10 may be scanned and a picture 35 of the tissue structure may be displayed by the display device 30.

In fig. 8 an apparatus as that shown in fig. 7 is used for examining the meat body or rib of a beef 32. However, in fig. 8 the probe 10 is not rotated, but ultrasonic vibrations are emitted and detected in an angular or conical pattern while the probe 10 is being moved gradually into and out from the meat body as indicated by an arrow 41. A picture 35 of the scanned tissue structure and of the meat/fat pattern may be displayed by the display device 30. Furthermore, the device 30 may display the fat/meat relationship or percentage.

**Claims**

1. A method of determining and/or evaluating quality characteristics of a body of meat material, said method comprising

   plunging at least one probe containing means for emitting mechanical vibrations into said body so as to position the vibration emitting means within said body,

   emitting mechanical vibrations within said body by the vibration emitting means,

   detecting mechanical vibrations emitted by the emitting means by a detecting means,

   generating detection signals in response to mechanical vibrations detected by the detecting means,

   determining and/or evaluating quality characteristics of said body on the basis of signals detected.

2. A method according to claim 1, wherein the detected signals are analog signals, which are transformed and converted into digital signals.

3. A method according to claim 1 or 2, wherein said body is part of an animal carcass.

4. A method according to any of the claims 1-3, wherein mechanical vibrations are detected by detecting means arranged within said at least one probe.

5. A method according to any of the claims 1-4, wherein the mechanical vibrations are ultrasonic vibrations.

6. A method according to any of the claims 1-5, wherein the mechanical vibrations emitted are directed transversely to the longitudinal direction of the probe containing the emitting means.

7. A method according to claim 6, wherein the mechanical vibrations are emitted substantially at right angles to the longitudinal direction of the probe.

8. A method according to any of the claims 1-7, wherein mechanical vibrations are emitted by the emitting means through an angle, the vertex of which is positioned at the emitting means.

9. A method according to claim 8, wherein said angle is defined in a plane extending through the longitudinal axis of the probe, which contains the emitting means.

10. A method according to any of the claims 1-9, wherein mechanical vibrations are emitted by the emitting means as a band-like beam along a plane extending through the longitudinal axis of the probe, which contains the emitting means.

11. A method according to any of the claims 6-10, wherein the emitting means are rotated about the longitudinal axis of the probe, which contains the emitting means, while mechanical vibrations are emitted and detected continuously or intermittently.

12. A method according to any of the claims 1-11, wherein mechanical vibrations are emitted and detected continuously or intermittently while said at least one probe is plunged into said body and moved axially in relation thereto.

13. A method according to any of the claims 1-11, wherein at least two probes arranged in spaced relationship are plunged into said body.

14. A method according to claim 13, wherein the emitting means are arranged in one of said probes, the remaining probe or probes each containing vibration detecting means.

15. A method according to any of the claims 1-14, wherein mechanical vibrations are emitted into said body by emitting means arranged outside said body.

16. A method according to any of the claims 1-15, wherein mechanical vibrations are detected by detecting means arranged outside said body.

17. A method according to any of the claims 1-16, wherein the frequency of the vibrations emitted is changed.

18. A method according to any of the claims 1-17, wherein the contour of a tissue interface in said body is determined by analyzing detection signals in response to detected ultrasonic vibrations reflected by said interface.

19. A method according to any of the claims 13-18, wherein quality characteristics are determined by measuring and analyzing the velocity of propagation of the vibrations, or the time for transmitting the vibrations from one probe to another.

20. A method according to any of the claims 13-19, wherein quality characteristics are determined by measuring and analyzing impedance of the material between two probes.

21. A method according to any of the claims 1-20, wherein quality characteristics are determined on the basis of analysis of intensity and/or frequency spectra of the vibration detected.

22. A method according to any of the claims 1-21, wherein duration of echoes and/or characteristics of echoes of the mechanical vibrations is/are detected, and the evaluation of quality characteristics being based thereon.

23. A method according to any of the claims 1-22, further comprising determining the thrust necessary for plunging the probe into said body and partly basing the evaluation of quality characteristics on the thrust determined.

24. A method according to any of the claims 1-23, further comprising measuring the temperature of the body of meat material at the location of at least one probe and taking the temperature measured into account when evaluating the quality characteristics.

25. A method according to any of the claims 1-24, wherein the depth of penetration of the emitting means and/or detecting means into the body of meat material is determined at one or more depths of penetration, the vibrations detected being interrelated to the determined depth of penetration.

26. A method according to any of the claims 6-25, wherein the rotational position of the probe or probes is continuously or intermittently determined and interrelated to the vibrations detected.

27. An apparatus for determining and/or evaluating quality characteristics of a body of meat material, said apparatus comprising
    at least one probe to be plunged into said body and including means for emitting mechanical vibrations,
    means for detecting mechanical vibrations emitted by the emitting means, and
    means for generating detection signals in response to mechanical vibrations detected by the detecting means.

28. An apparatus according to claim 27, wherein the detecting means are arranged within said at least one probe.

29. An apparatus according to claim 27 or 28, wherein the vibration emitting means comprise an ultrasonic transducer.

30. An apparatus according to any of the claims 27-29, wherein said at least one probe has a free end defining a pointed end or cutting edge.

31. An apparatus according to any of the claims 27-29, wherein the means for emitting mechanical vibrations are positioned in the probe and adapted to direct generated vibrations transversely to the longitudinal axis of the probe containing the emitting means.

32. An apparatus according to any of the claims 27-31, further comprising means for rotating the emitting means about the longitudinal axis of the probe containing the emitting means.

33. An apparatus according to claim 32, further comprising means for interrelating the rotational position of the probe to the vibrations detected.

34. An apparatus according to any of the claims 30-33, further comprising means for determining the thrust used for forcing the pointed end or cutting edge of the probe into the body of meat material.

35. An apparatus according to any of the claims 27-34 and comprising at least two probes arranged in transversely spaced relationship.

36. An apparatus according to claim 35, wherein the emitting means are arranged in one of said probes, the other probe or probes each including detecting means.

37. An apparatus according to any of the claims 27-36, further comprising means for determining the depth of penetration of the probe or probes in relation to said body.

**38.** An apparatus according to any of the claims 27-37, further comprising means for processing and/or registering said detection signals.

**39.** An apparatus according to claim 38, wherein the processing and registering means comprise transforming and converting means for converting analog detection signals received into digital signals.

**40.** An apparatus according to claim 38 or 39, wherein said processing means comprise neural networks.

Fig. 1

Fig. 2a

Fig. 2b

Fig. 3

Fig. 4

Fig. 5

Fig. 6

Fig. 7

Fig. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SOVIET INVENTIONS ILLUSTRATED, Derwent Publications Ltd., LONDON, GB Section Electrical, Week C08, 02 April 1980, abstract no. B7426C/08, class R16; & WPI Database, Accession No. 80-B7426C; & SU-A-665261 (AS BIOL PHYS INST) 30 May 1979 * the whole document* | 1, 3-7, 10, 12-16, 19, 21 27-31, 35, 36, 38 | G01N33/12 A22B5/00 G01N29/18 |
| Y | * idem * | 2, 8, 9, 11, 18, 22, 23 25, 26, 32-34, 37, 39 | |
| A | * idem * | 17, 20, 24 | |
| Y | DE-A-3619292 (H. RUMP) * the whole document * | 2, 22, 39 | |
| A | * idem * | 1, 3, 38 | |
| D,Y | US-A-4756313 (R. TERWILLIGER) * column 3, line 5 - column 5, line 24 * * figure 1 * | 8, 9, 11, 26 32, 33 | TECHNICAL FIELDS SEARCHED (Int. Cl.5) |
| D,A | * idem * | 1, 25, 37 | G01N A22B |
| D,Y | US-A-4785817 (J.R. STOUFFER) | 18 | |
| A | * the whole document * | 1, 2, 38-40 | |
| Y | WO-A-8801742 (G. JOHNSTON) * page 3, paragraph 5 - page 4, paragraph 4 * * figure 1 * | 23, 25, 34, 37 | |
| A | * idem * | 1, 27 | |
| A | GB-A-2213263 (AGRICULTURAL & FOOD RESEARCH COUNCIL) * page 8, paragraph 3 - page 12, paragraph 3 * * figures 1, 2 * | 1-3, 13, 15, 16, 19, 21 39 | |

The present search report has been drawn up for all claims

| Place of search | Date of completion of the search | Examiner |
|---|---|---|
| BERLIN | 10 OCTOBER 1991 | JOHNSON, K |

European Patent
Office

EUROPEAN SEARCH REPORT

Application Number

EP    91 61 0009
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PATENT ABSTRACTS OF JAPAN vol. 10, no. 30 (P-426)(2087) 05 February 1986, & JP-A-60 181606 (YOSHIO TAKEMORI) 17 September 1985, * the whole document * | 1, 3, 12, 22, 23, 25, 34 37, 38 | |
| A | EP-A-337661 (J. WILSON) * column 5, line 35 - column 7, line 49 * | 1, 18, 27, 38, 40 | |
| A | EP-A-321981 (SLAGTERIERNES FORSKNINGSINSTITUT) * page 8, lines 12 - 55 * | 1, 18, 27, 38, 40 | |
| A | FR-A-2605096 (F. CHAGNEAU ET AL) * pages 1 - 2; figures 2, 3 * | 1, 25, 27, 37 | |
| A | ULTRASONICS. vol. 21, no. 5, September 1983, GUILDFORD GB pages 226 - 233; D.J. HAUMSCHILD ET AL: "An ultrasonic Bragg scattering technique for the quantitative characterization of marbling in beef" * page 229, right-hand column, paragraph 3 - page 230, left-hand column, paragraph 3 * | 1, 27, 38, 40 | TECHNICAL FIELDS SEARCHED (Int. Cl.5) |

The present search report has been drawn up for all claims

| Place of search | Date of completion of the search | Examiner |
|---|---|---|
| BERLIN | 10 OCTOBER 1991 | JOHNSON, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)